## Europäisches Patentamt
## European Patent Office
## Office européen des brevets

(19)

(11) Veröffentlichungsnummer: **0 336 976**
**A1**

(12) ## EUROPÄISCHE PATENTANMELDUNG
Veröffentlicht nach Art. 158 Abs. 3 EPÜ

(21) Anmeldenummer: **88908418.2**

(22) Anmeldetag: **19.08.88**

Daten der zugrundeliegenden internationalen Anmeldung:

(86) Internationale Anmeldenummer:
**PCT/SU88/00162**

(87) Internationale Veröffentlichungsnummer:
**WO89/02745 (06.04.89 89/08)**

(51) Int.Cl.³: **A 61 K 37/02**

(30) Priorität: **02.10.87 SU 4312985**

(43) Veröffentlichungstag der Anmeldung:
**18.10.89 Patentblatt 89/42**

(84) Benannte Vertragsstaaten:
**AT BE CH DE FR GB IT LI NL SE**

(71) Anmelder: **VSESOJUZNY KARDIOLOGICHESKY NAUCHNY TSENTR AKADEMII MEDITSINSKIKH NAUK SSSR**
**3 Cherepkovskaya ul, 15a,**
**Moscow, 121552(SU)**

(72) Erfinder: **GUSEV, Evgeny Ivanovich**
**Leninsky pr., 156-277**
**Moscow, 117571(SU)**

(72) Erfinder: **ZAVALISHIN, Igor Alexeevich**
**ul. Komarova, 1-9**
**Moskovskaya obl. Dedovsk, 143530(SU)**

(72) Erfinder: **DEMINA, Tatyana Leonidovna**
**ul. Moskovskaya, 7/1-33**
**Moskovskaya obl. Khimki, 141400(SU)**

(72) Erfinder: **NEVSKAYA, Olga Mikhailovna**
**Bykovskoe shosse, 30-64**
**pos. Malakhovka Moskovskaya obl., 140090(SU)**

(72) Erfinder: **TITOV, Mikhail Ivanovich**
**ul. Osennaya, 2-117**
**Moscow, 121609(SU)**

(72) Erfinder: **BESPALOVA, Zhanna Dmitrievna**
**ul. Kuntsevskaya, 1/5-229**
**Moscow, 121351(SU)**

(72) Erfinder: **VINOGRADOV, Valentin Antonovich**
**Khoroshevskoe shosse, 34-38**
**Moscow, 123007(SU)**

(72) Erfinder: **POLONSKY, Vladimir Markovich**
**ul. Krasina, 14-25**
**Moscow, 123056(SU)**

(74) Vertreter: **von Füner, Alexander, Dr. et al,**
**Patentanwälte v. Füner, Ebbinghaus, Finck Mariahilfplatz 2 & 3**
**D-8000 München 90(DE)**

(54) **ARZNEIMITTELZUBEREITUNG ZUR BEHANDLUNG DER MULTIPLEN SKLEROSE UND DER SEITLICHEN AMYOTROPHEN SKLEROSE.**

(57) Die Erfindung bezieht sich auf das Gebiet der Medizin.
Arzneimittel für Behandlung der multiplen Sklerose und der amyotrophischen Lateralsklerose setzt sich aus einem Wirkstoff, einem Peptid folgender Struktur:
Tyr-D-Ala-Gly-Phe-Leu-Arg
und einem pharmazeutischen Verdünnungsmittel zusammen.

ARZNEIMITTEL FUR BEHANDLUNG DER MULTIPLEN
SKLEROSE UND DER AMYOTROPHISCHEN LATERALSKLEROSE

Technisches Gebiet

Die vorliegende Erfindung bezieht sich auf das Gebiet der Medizin, und insbesondere auf ein neues Arzneimittel für Behandlung der multiplen Sklerose und der amyotrophischen Lateralsklerose.

Vorhergehender Stand der Technik

Gegenwärtig schliesst die Behandlung der multiplen Sklerose die Einwirkung auf die führenden pathogenetischen Mechanismen sowie die Verwendung von symptomatischen Mitteln ein. In der Phase der Verschlimmerung der multiplen Sklerose werden Kortikosteroidhormone, Mittel die die Erzeugung von Endogen-Interferon stimulieren (Proper-mil, Prohidiosan), Desoxyribonuklease und Interferon (Hughes R.A.S. Immunological treatment of multiple sclerosis, j. Neurol., I983, 230, 2, p. 73-80) verordnet. Man verwendet Präparate, die den Gewebemetabolismus beeinflussen (Nootropil, Enzephabol, Zerebrolysin), Korrekturmittel der Mikrozirkulation (Kurantil, Trental), antiaggregante Mittel (Azetylsalizylsäure, Phytin, Gemisch aus Kalzium- und Magnesiumsalzen der Inositphosphorsäuren), immunomodulierende Mittel (T-Aktivin, Thymalin) (Gusev E.I., Arion V.Ja, Aristova R.A und andere, Erfahrungen bei der Anwendung von T-Aktivin für Patienten mit multipler Sklerose (Opyt primenenia T-aktivina u bolnykh rasseyannym sklerosom). Journal für Neuropathologie und Psychiatrie namens Korsakow, I983, (Moskau), "Medizin", 84, S. I68-I73).

Zur Zeit hat die laterale amyotrophische Lateralsklerose keine effektive äthyologische und pathogenetische Behandlung. Der zum Einsatz kommende Komplex von Präparaten schliesst Antioxydationsmittel und Substanzen ein, die den Eiweisstoffwechsel im zentralen Nervensystem aktivieren, sowie symptomatische Mittel, die den Muskeltonus, die Atmung und die Herztätigkeit regulieren (Behandlung von Nervenkrankheiten. (Letchenie nervnykh zabolevany). Unter Redaktion von V.K.Viderholt, I984, (Moskau),

- 2 -

"Medizin", S. 336-339).

Die gegenwärtig durchzuführende Behandlung der multiplen Sklerose und der amyotrophischen Lateralsklerose zeichnet sich durch eine geringe Effektivität aus und beugt nicht die Weiterentwicklung und Aktivierung des Prozesses vor. Zu gleicher Zeit rufen die für die Behandlung der multiplen Sklerose einzusetzenden Kortikosteroide und Interferon unerwünschte Nebenreaktionen hervor.

In der Literatur ist ein Peptid folgender Struktur: Tyr-D-Ala-Gly-Phe-Leu-Arg (Chavkin, Goldstein, Proc. Natl. Acad. Sci, USA, 1981,78 p. 6543-6547) ohne Hinweis auf das mögliche Gebiet seiner Anwendung beschrieben. Später wurde die antiulzeröse Wirkung dieses Peptides nachgewiesen (PCT-Anmeldung SU 83/00039).

Gegenwärtig liegen Angaben über die günstige Wirkung dieses Peptids auf die Entwicklung der experimentellen Pankreonekrose vor (Georgadze A.K., Permjakov N.K., Pennin V.A. und andere). Vergleichende Einschützung der Einwirkung von Kontrikal, 5-Fluorurazyl und des neuen einheimischen Präparates, Dalargins auf den Verlauf der experimentellen Pankreatitis, (Farmakologija und toksikologija 1986, (Moskau), "Medizin", Nr. 6, S. 66-70), über seine immunomodulierende Wirkung (Nekrasov E.L., Vinogradov V.A., Opioide Peptide als Regulatoren des Immunitätsystems (Opioide peptide kak regulatory sistemy immuniteta), Bulletin VKHTs der Akademie für medizinische Wissenschaften der UdSSR, 1987, (Moskau), "Medizin", Nr. I, S. 3-I0.) Die Anwendung des genannten Peptids für die Behandlung organischer Beschädigungen des Nervensystems, insbesondere der multiplen Sklerose und der myotrophischen Lateralsklerose, ist unbekannt.

Offenbarung der Erfindung

Das angemeldete Arzneimittel ist neu und in der Literatur nicht beschrieben.

Der Erfindung liegt die Aufgabe zugrunde, ein neues Arzneimittel für die Behandlung der multiplen Sklerose und der amyotrophischen Lateralsklerose zu entwickeln, das eine hohe Effektivität aufweist, die Übertragung eines Nervenimpulses verbessert und keine unerwünschten Nebenreaktionen hervorruft.

Die Aufgabe wurde dadurch gelöst, dass das Arzneimittel für die Behandlung der multiplen Sklerose und der amyotrophischen Lateralsklerose, das einen Wirkstoff und ein pharmazeutisches Verdünnungsmittel enthält, erfindungsgemäss, als Wirkstoff ein Peptid folgender Struktur aufweist:

Tyr-D-Ala-Gly-Phe-Leu-Arg.

Das angemeldete Präparat kann in verschiedenen Arzneiformen (Injektionslösung, intranasale Aerosole, Zöpfchen, Tabletten und anderes mehr) angewendet werden. Die bevorzugte Form der Anwendung des angemeldeten Präparates ist die Form von Injektionslösungen bei einer einmaligen Dosis des Wirkstoffes von I bis 5 mg. Als pharmazeutisches Verdünnungsmittel enthält das Präparat vorzugsweise ein Verdünnungsmittel, 0,9%ige wässerige Natriumchloridlösung.

Das Präparat in Form von Tabletten enthält vorzugsweise den Wirkstoff in einer Menge von IO bis I5 mg je eine Tablette. Als pharmazeutische Trägersubstanz für Tabletten enthält es vorzugsweise Stärke, Glukose oder Laktose.

Das Präparat in Form eines intranasalen Aerosols enthält vorzugsweise den Wirkstoff in einer Menge von I bis 5 mg in einer einmaligen Dosis. Als pharmazeutisches Verdünnungsmittel kann das Präparat eine 0,9%ige wässerige Natriumchloridlösung aufweisen, der pharmazeutisch annehmbare Detergentiummittel hinzugefügt werden können.

Das vorgeschlagene Präparat fördert bei der Behandlung der multiplen Sklerose, die unter Anwendung der Basistherapie erfolgt, die klinische Verbesserung des Zustandes der Mehrheit von Patienten, die positive Dynamik aller zerstörten Funktionen des Nervensystems und, was besonders wichtig ist, die Verringerung eines erhöhten Muskeltonus, die Verringerung des Ausprägegrades der zerebralen Symptome und die Verbesserung des funktionellen Zustandes der Leitbahnen des Hirnstammes.

- 4 -

Bei Behandlung der amyotrophischen Lateralsklerose mit dem vorgeschlagenen Präparat unter Anwendung der Basistherapie erfolgt die Vorbeugung der sich verschlimmernden Entwicklung der Erkrankung bei den meisten Patienten, eine positive Veränderung im neurologischen Status der Patienten, die Verringerung des Ausprägegrades depressiver Reaktionen.

Das Präparat besitzt eine niedrige Toxizität ($LD_{50}$ beträgt 520 mg/kg), eine geringe einmalige Dosis und geringe Dosis für die gesamte Behandlungsdauer sowie einen umfangreichen therapeutischen Wirkungsbereich.

Beste Ausführungsvariante der Erfindung

Das erfindungsgemässe Präparat für die Behandlung der multiplen Sklerose und der myotrophischen Lateralsklerose wurde in einer Klinik an Menschen erprobt.

Bei der multiplen Sklerose erfolgte die Behandlung mit dem angemeldeten Präparat unter Anwendung der Basistherapie, die die Vitamine der B-Gruppe, Glutaminsäure, Nikotinsäure, Zerebrolysin, Midokalm, und Essentiale einschliesst. Das erfindungsgemässe Präparat wurde den Patienten intramuskulär zu je I bis 5 mg des Wirkstoffes, der in I ml einer physiologischen Lösung aufgelöst wird, zweimal pro Tag innerhalb von 5 Tagen eingeführt.

Bei der amyotrophischen Lateralsklerose erfolgte die Behandlung mit dem erfindungsgemässen Präparat unter Anwendung der Basistherapie, die Zerebrolysin, Retobolyl, Vitamine der B-Gruppe, Vitamin E, Anticholinesterase-Mittel, Myorelaxationsmittel und Biostimulatoren einschliesst. Das angemeldete Präparat wurde den Patienten intravenös oder intramuskulär zu je I bis 5 mg des Wirkstoffes aufgelöst, in I ml physiologischer Lösung, zweimal am Tage innerhalb von I4 Tagen eingeführt. Später wurden die Behandlungen mit dem Präparat mit Unterbrechungen von IO Tagen I bis 3 mal durchgeführt.

Bei der Behandlung der multiplen Sklerose wurde die Wirkung des erfindungsgemässen Präparates mit der Wirkung von T-Aktivin verglichen, das unter Anwendung der gleichen wie bei der Behandlung mit dem angemeldeten Präparat Basistherapie eingeführt wurde. Das T-Aktivin führte man den Patienten intramuskulär einmal pro Tag zu je I ml I,0%iger Lösung innerhalb von 5 Tagen ein. Nach einer 7-tägigen Unterbrechung führte man das T-Aktivin in der gleichen Dosis während 2 Tage ein.

Bei der Behandlung der multiplen Sklerose mit dem erfindungsgemässen Präparat und mit T-Aktivin verwendete man neben einer klinischen Analyse der neurologischen Symptome auch eine Methode der Registrierung von kurzlatenten erzeugten Hörpotentialen auf die akustische Stimulation. Die Dynamik der neurologischen Symptome bewertete man in Punkten nach der Kurzkschen Skale. Man führt die Analyse der Punkten-Summe nach der Skale des neurologischen Defizits durch sowie nach der Skale der Invalidisierung (sich verschlimmernde Entwicklung) unter Berücksichtigung der sozialen Adaptation des jeweiligen Patienten durch. Bei der Untersuchung der kurzlatenten erzeugten Hörpotentiale erfolgte die Summierung von 3000 Antworten und es wurden 7 Komponenten registriert, die den funktionellen Zustand folgender Pegel eines Höranalysators widerspiegeln: I - Schneke des Hörnervs, II - Kerne des Cochlearkomplexes, III- Nucleus der oberen Olive, IV - Nucleus der lateralen Schleife, V - Kerne der Höcker der Vierhügelplatte, VI - Kerne mittlerer Kniehöcker, VII - Kerne Auditus kortex. Alle Komponenten wurden nach den Werten der Zwischenpeakintervalle und den Relationen der Peakamplituden analysiert. Die Kontrollgruppe für die Untersuchung der kurzlatenten erzeugten Hörpotentiale bildeten I2 praktisch gesunde Personen.

Die Untersuchung der Patienten erfolgte vor der Verordnung der Präparate, in 5 und in I4 Tagen nach dem Beginn ihrer Verabreichung.

Hervorzuheben ist, dass bei den Patienten, denen das erfindungsgemässe Präparat und das T-Aktivin verordnet wurden, innnerhalb der letzten zwei Monate keine Dynamik im neurologischen Status nachgewiesen wurde. Das gab die Möglichkeit, die Verbesserung des Zustandes als Effekt von der Verordnung der Präparate zu betrachten.

Die Hauptgruppe der Patienten, die das erfindungsgemässe Präparat verabreicht bekamen, bestand aus 24 Personen (6 Männer und I8 Frauen) im Alter von 2C bis 49 Jahren mit zerebrospinaler Form der multiplen Sklerose. Die Dauer der Erkrankung betrug von 2 bis 7 Jahren. Das erfindungsgemässe Präparat führe man den Patienten intramuskulär zu je I bis 2 mg zweimal am Tage innerhalb von 5 Tagen ein. Die Gruppe der Patienten, die das T-Aktivin bekam, bestand aus I4 Personen (6 Männer und 8 Frauen) im Alter von 23 bis 47 Jahren mit zerebrospinaler Form der multiplen Sklerose. Die Dauer der Erkrankung betrug von 2 bis 7 Jahren.

Das T-Aktivin führte man den Patienten intramuskulär zu je I ml 0,I%iger Lösung einmal pro Tag innerhalb von 5 Tagen ein.

Infolge der Behandlung der multiplen Sklerose mit dem erfindungsgemässen Präparat wurde eine bedeutende klinische Verbesserung bei 89% der Patienten beobachtet. In der Gruppe insgesamt kam das zum Ausdruck in der Verringerung der Summe der Punkte nach der Skale des neurologischen Defizits um 2,9 und um 3,8 Punkte in 5 und in I4 Tagen nach dem Beginn der Behandlung. Der Index nach der Skale der Weiterentwicklung der Erkrankung sank in I4 Tagen nach dem Beginn der Behandlung um I,3 Punkte. Die Angaben über die Effektivität der Behandlung mit dem erfindungsgemässen Präparat in Abhängigkeit vom Charakter des Verlaufs und des Schweregrades der multiplen Sklerose sind in der Tabelle I angeführt. Ein bedeutender klinischer Effekt wurde bei einer einzigen Kranken festgestellt, die im akuten Zustand der multiplen Sklerose eingeliefert wurde: zum I5. Tag verringerte sich die Summe der Punkte nach der Skale des neurologischen Defizits und der Index nach der Skale der Weiterentwicklung der Krankheit um IO und um 3 Punkte, was im Endergebnis den Eintritt einer Remission bedeutete.

Eine nachweisbare Verringerung der Summe der Punkte nach der Skale des neurologischen Defizits wurde bei Patienten mit remittierendem Verlauf im Zustand der Remission (um 3,I Punkte) und bei der sekundären Verschlimmerung des Prozesses (um 6,3 Punkte) beobachtet. Im letzten Fall wurde auch eine nachweisbare Senkung des Indexes nach der Skale der sich verschlimmernden Entwicklung (um 2,4 Punkte) festgestellt, der therapeutische Effekt war verzögert und wurde hauptsächlich nach der Beendigung der Einführung des erfindungsgemässen Präparates (am I5. Tag seiner Verabreichung) beobachtet. Die Verbesserung des neurologischen Status unter Einwirkung des angemeldeten Präparates wurden bei Patienten sowohl mit leichtem als auch mit mittlerem Schweregrad der multiplen Sklerose anchgewisen, wobei im letzten Fall der Effekt ausgeprägter war und bezog sich sowohl auf die Summe der Punkte nach der Skale des neurologischen Defizits als auch auf den Index nach der Skale der sich verschlimmernden Entwicklung (Verringerung am 6. Tag um 4,3 und I,2 Punkte). Die von dem erfindungsgemässen Präparat hervorgerufene Verbesserung des klinischen Zustandes kam in der Verringerung der vorwiegend pyramidenartigen Sensusstörungen sowie in der Verbesserung der Funktion des Hirnstammes, des Kleinhirns und der Beckenorgane zum Vorschein.

- 8 -

Tabelle I

Dynamik des neurologischen Zustands der Patienten, die das erfindungsgemässe Präparat bekamen, unter Berücksichtigung des Verlaufstyps

| Gruppe der Patienten | Anzhal der Patienten | Vor der Behandlung | |
|---|---|---|---|
| | | Gesamtpunktenzahl nach der Skala des neurologischen Defizits | Index nach der Skale der sich verschlimmernden Entwicklung |
| I | 2 | 3 | 4 |
| Nach dem Verlauf I. Remittierend | | | |
| a) Verschlimmerung | I | I5 | 5 |
| b) Remission | 9 | I3,8+I,I | 4,3+0,8 |
| c) sekundäre Verschlimmerung | 7 | I5,I+I,5 | 5,9+I,I |
| 2. Progredient | 7 | I3,7+I,3 | 4,7+I,3 |
| Nach dem Schweregrad | | | |
| I. Leichter Grad | I2 | II,7+I,0 | 3,7+0,4 |
| 2. Mittlerer Grad | I2 | IO,7+I,4 | 6,I+0,3 |

Fortsetzung der Tabelle I

| Gruppe der Patienten | Am 6. Tag | | Am I5. Tag | |
|---|---|---|---|---|
| | Gesamtpunktenzahl nach der Skale des neurologischen Defizits | Index nach der Skale der sich ver schlimmernden Entwicklung | Gesamtpunktenzahl nach der Skala des neurologischen Defizits | Index nach der Skale der sich verschlimmernden Entwicklung |
| I | 5 | 6 | 7 | 8 |
| Nach dem Verlauf I. Remittierend a) Verschlimmerung | IO | 3 | 5 | 2 |

| I | 5 | 6 | 7 | 8 |
|---|---|---|---|---|
| b) Remission | IO,7±I,0[*] | 3,3±0,5 | II,0+09[*] | 3,4±0,5 |
| c) sekundäre Verschlimmerung | I3,0±I,2 | 4,8±2,0 | 9,8±I,0[*] | 3,5±0,4 |
| Progredient | I2,0±I,2 | 4,I±I,9 | II,7±I,I | 4,0±I,5 |
| Nach dem Schweregrad | | | | |
| I. Leichter Grad | IO,2±I,I | 3,I±0,4 | 8,6±0,9[*] | 2,8±0,5 |
| 2. Mittlerer Grad | I2,4±I,3[*] | 4,9±0,3 | I2,2±I,I[*] | 4,4±0,6[*] |

[*] - Differenz zum Ausgangsstand ist zuverlässig in der Wertenhöhe von 95% (P ∠ 0,05).

Neben der klinischen Verbesserung wurde auch die Fähigkeit des erfindungsgemässen Präparates festgestellt, den funktionellen Zustand der Leitungsbahnen des Hirnstammes zu verbessern, der mit Hilfe der Methode der Registrierung kurzlatenter erzeugter Hörpotentiale nachgewiesen wurde.

Vor der Verordnung des Präparates waren bei Patienten Änderungen der kurzlatenten erzeugten Hörpotentiale in Form der Störungen der Kurvenform, der Vergrösserung von Zwischenpeakintervalle, der Verringerung der Peakamplitude und der Veränderung der Amplitudenrelation zu verzeichnen. Die Angaben über den Einfluss auf die Hauptkennziffern, die unter Anwendung dieser Methode bewertet wurden, sind in der Tabelle 2 angeführt.

So wurde auf der Grundlage der Behandlung mit dem erfindungsgemässen Präparat die Verringerung der interhemisphärischen Assymetrie, die teilweise Wiederherstellung der Kurvenform der kurzlatenten erzeugten Hörpotentiale festgestellt. Der maximale Effekt wurde am I5. Tag nach dem Beginn der Behandlung mit dem erfindungsgemässen Präparat, das heisst am IO. Tag nach seiner Absetzung, beobachtet, wenn eine zuverlässige Vergrösserung des Verhältnisses der Amplitude V des Peaks zum Peak I zu verzeichnen war.

Infolge eines 5-tägigen Behandlungskursus mit T-Ak-

tivin wurde ein unwesentlicher klinischer Effekt erreicht, der in einer unzuverlässigen Verringerung der Gesamtpunktzahl nach der Skale des neurologischen Defizits (um 0,8 insgesamt in der Gruppe) zum Ausdruck kam. Es verringerten sich hauptsächlich die Pyramidensymptome sowie der Ausprägegrad der Merkmale der Schädigung des Hirnstammes. Eine solche Dynamik wurde in der Hauptsache in einer Gruppe von Patienten mit progredientem Verlauf der multiplen Sklerose beobachtet, aber auch hier war sie statistisch nicht bedeutungshaltig (Angaben sind in der Tabelle 2 angeführt).

Bei der Analyse des neurologischen Status unter Berücksichtigung des Schweregrades des Verlaufs wurde eine unwesentliche positive Dynamik bei Patienten sowohl mit leichtem Grad als auch mit mittlerem Schweregrad beobachtet. In I4 Tagen nach dem Beginn der Behandlung mit T-Aktivin wurde eine ausgeprägtere Verbesserung des neurologischen Status festgestellt. Insgesamt für die Gruppe verringerte sich die Gesamtpunktenanzahl nach der Skale des neurologischen Defizits um 2,4 Punkte und der Index nach der Skale der sich verschlimmernden Entwicklung um 0,7 Punkte. Die Senkung hatte den Charakter einer Tendenz und war statistisch gesehen, nicht bedeutungshaltig.

Tabelle 2

Einfluss des erfindungsgemässen Präparates auf die relativen Kennziffern der Methode der Registrierung kurzlatenter erzeugter Hörpotentiale auf akustische Stimulation

| Lfd. Nr. | Zeit der Untersuchung | Differenzen der Latenzwerte | | |
|---|---|---|---|---|
| | | III Peak-I Peak | V Peak-II Peak | V Peak-I Peak |
| I | 2 | 3 | 4 | 5 |
| I. | Vor Behandlung | 2,2I±0,07 | 2,49±0,I4 | 4,70±0,I4 |
| 2. | Am 6. Tag | 2,22±0,05 | 2,28±0,I3 | 4,50±0,II |
| 3. | Am I5. Tag | 2,20±0,06 | 2,I0±0,I3[*] | 4,30±0,I2[*] |

[*] - Differenz zum Ausgangsstand ist in der Wertenhöhe von 95% (P < 0,05) zuverlässig.

Fortsetzung der Tabelle 2

| Lfd. Nr. | Verhältnis der Amplituden | | |
| --- | --- | --- | --- |
| | II Peak-I Peak | V Peak - II Peak | V Peak - I Peak |
| I | 6 | 7 | 8 |
| I. | 0,75±0,I8 | 0,7I±0,I7 | 0,4I±0,II |
| 2. | 0,84±0,I9 | 0,94±0,2I | 0,64±0,I6 |
| 3. | 0,67±0,I5 | I,53±0,33[x] | 0,88±0,23 |

Tabelle 3

Dynamik des neurologischen Status von Petienten, die T-Aktivin bekamen, unter Berücksichtigung des Typs des Verlaufs

| Lfd. Nr. | Gruppen der Petienten | Anzahl der Patienten | Vor der Behandlung | |
| --- | --- | --- | --- | --- |
| | | | Gesamtpunktenzahl nach der Skale des neurologischen Defizits | Index nach der Skale der sich verschlimmernden Entwicklung |
| I | 2 | 3 | 4 | 5 |
| Nach dem Verlauf | | | | |
| I. Remittierend | | | | |
| a) Verschlimmerung | | 2 | I0,0±I,2 | 3,5±0,5 |
| b) Remission | | 4 | I3,5±I,2 | 4,3±0,4 |
| c) sekundäre Verschlimmerung | | 2 | I7,5±I,8 | 6,5±2,5 |
| 2. Progredient | | 6 | I3,7±I,3 | 4,3±I,0 |
| Nach dem Schweregrad | | | | |
| I. Leichter Grad | | 9 | II,0±I,0 | 3,7±0,4 |
| 2. Mittlerer Grad | | 5 | I8,4±I,7 | 6,0±I,2 |

- I2 -

| Lfd. Nr. | Gruppen der Patienten | Am 6. Tag | | Am I5. Tag | |
|---|---|---|---|---|---|
| | | Gesamt- punkten- anzahl nach der Skale des neu- rologischen Defizits | Index nach der Skale der sich verschlim- mernden Ent- wicklung | Gesamt- punkten- anzahl nach der Skale des neu- rologischen Defizits | Index nach der Skale der sich verschlim- mernden Ent- wicklung |
| I | 2 | 6 | 7 | 8 | 9 |
| Nach dem Verlauf | | | | | |
| I. Remittierend | | | | | |
| a) Verschlim- merung | | 9,5±I,0 | 3,5±0,5 | 5,5±0,8 | 2,5±0,5 |
| b) Remission | | I3,0±I,3 | 4,3±0,4 | II,0±I,2 | 3,5±0,5 |
| c) sekundäre Verschlim- merung | | I6,5±I,8 | 6,5±2,5 | I4,5±I,5 | 6,0±3,0 |
| 2. Progredient | | I2,5±I,5 | 4,2±0,9 | II,3±I,3 | 3,7±I,3 |
| Nach dem Schwe- regrad | | | | | |
| I. Leichter Grad | | I0,3±I,I | 3,7±0,4 | 7,9±0,8[*] | 2,9±0,6 |
| 2. Mittlerer Grad | | I7,2±I,9 | 5,8±I,4 | I6,2±I,7 | 5,4±I,7 |

[*] - Differenz zum Ausgangsstand ist in der Wertenhöhe von 95% (P < 0,05) zuverlässig.

Die Effektivität des T-Aktivins bei Patienten mit leichtem und mittlerem Schweregrad der multiplen Sklerose hat sich wesentlich unterschieden. Wenn das T-Aktivin bei den Patienten mit leichtem Schweregrad die Gesamtpunktenzahl nach der Skale des neurologischen Defizits (um 3,I Punkte) zuverlässig herabsetzte, so war der Effekt bei dem mittleren Schweregrad des Verlaufs der multiplen Sklerose unwesentlich. Nach einer Kur mit dem T-Aktivin wurde eine klinische Verbesserung bei 56% der Patienten insgesamt festgestellt. Bei der Untersuchung der kurzlatenten erzeugten Hörpotentiale bei der Behandlung mit T-Aktivin wurde ein unzuverlässiges Ansteigen

- 13 -

der Amplitude des Peaks III festgestellt und eine Tendenz zur Vergrösserung des Verhältnisses der Amplitude des Peaks III zum Peak I beobachtet, was die Stimulierung der Strukturausbildungen auf der Ebene der Varolsbrücke des Hirnstammes widerspiegelt.

Hierdurch wurde auf Grund der durchgeführten Untersuchungen nachgewiesen, dass der klinische Effekt nach einer kurzen Behandlung mit T-Aktivin vom Schweregrad, vom Typ des Verlaufs der multiplen Sklerose und von der Dauer des Bestehens eines organischen Defizits abhängig war. Eine spürbarere Verbesserung wurde bei Patienten mit der grössten Dauer der Erkrankung (bis zu 3 Jahren) mit einem relativ niedrigen Grad des neurologischen Defizits und vorwiegend (nach den Angaben der Beobachtung am 15. Tag) bei Patienten im Zustand der Remission bei einem remittierenden Verlauf erzielt. Die Verbesserung betraf hauptsächlich die Pyramidensymptome und die Anzeichen der Beschädigung des Hirnstammes. Das T-Aktivin erwies keinen wesentlichen Einfluss auf den Muskeltonus und auf zerebrale Kleinhirnstörungen. Der Einfluss des T-Aktivins auf den funktionellen Zustand der Leitbahnen des Hirnstammes - nach Angaben der Methode der Registrierung kurzlatenter erzeugter Hörpotentiale - kam in einer nichtspezifischen Stimulierung der mesodienzephalen Strukturen zum Ausdruck, war kurzfristig und widerspiegelt offensichtlich hauptsächlich die Prozesse der nichtspezifischen Stimulation der Adaptation- und Anpassungsreaktionen eines Organismus.

Die Behandlung der multiplen Sklerose mit Hilfe des erfindungsgemässen Präparates hatte eine Reihe von Vorteilen im Vergleich mit der Behandlung mit T-Aktivin: klinische Verbesserung wurde öfters, in 89% (56% bei Behandlung mit T-Aktivin) beobachtet und sie war von dem Schweregrad und der Dauer der Erkrankung nicht abhängig; es wurde eine positive Dynamik seitens aller zerstörten Funktionen des Nervensystems (Verringerung der Pyramiden- und Sensorstörungen, Verbesserung der Funktionen des Hirnstammes, Kleinhirns und der Beckenorgane) nachgewie-

- I4 -

sen. Besonders wertvolle Eigenschaften des erfindungsgemässen Präparats, die für das T-Aktivin nicht kennzeichnend sind, bestehen in seiner Fähigkeit, den erhöhten Muskeltonus herabzusetzen, den Ausprägegrad von Kleinhirnsymptome zu verringern und den funktionellen Zustand der
Leitbahnen des Hirnstammes zu verbessern.

Bei der Behandlung der amyotrophischen Lateralsklerose wurde die Wirkung des erfindungsgemässen Präparates,
das man unter Anwendung der allgemeingültigen konventionellen Behandlung durchführte, die Zerebrolysin, Retabolyl, Vitamine der B-Gruppe, Vitamin E, Anticholinesterasemittel, Myorelaxantien, Biostimulatoren einschliesst,
mit der Wirkung dieses eines Komplexes von Medikamenten
verglichen.

Das klinische Bild war bei allen Patienten für die
amyotrophische Lateralsklerose typisch und zeichnete
sich durch Symptome der Verbindung der Schädigung anterotuberculum, bulbärrer Kernstrukturen und Pyramidenstrukturen, darunter supranuklearer Leiter aus.

Auf der Grund eines klinisch-anatomischen Unterschiedes, und zwar des Vorherrschens der Schädigungen
der anterotuberculum, bulbären Kernstrukturen oder Pyramidenleiter sowie der Anfangsanzeichen der Krankheit,
was insgesamt einem bestimmten anatomotopographischen
Stand des zentralen Nervensystems entspricht, wurden
folgende Formen der Erkrankung ausgesondert: cervicothoracius, lumbosacralis, bulbär, hohe und die sogenannte
poliomyelitische Variante.

Die Hauptgruppe von Patienten, die auf der Grundlage
der allgemeingültigen nichtspezifischen Behandlung das
beanspruchte Präparat verabreicht, bestand aus 62 Patienten mit amyotrophischen Lateralsklerose (39 Männer und
23 Frauen) im Alter von 2I bis 65 Jahren mit einer Dauer
der Erkrankung von 3 Monaten bis zu 4,5 Jahren. Bei 28
Patienten war generalisierte Form, bei I4 Patienten -
cervicothoracius, bei 7 - lumbosacralis, bei I2 - bulbäre Form und bei I Patienten - hohe Form der amyotrophischen Lateralsklerose.

- I5 -

Das erfindungsgemässe Präparat führte man den Patienten intramuskulär oder intravenös je I bis 2 mg zweimal am Tage innerhalb von I4 Tagen ein. Notwendigenfalls wurde der Behandlungskursus mit dem Präparat wiederholt.

Patientengruppe mit der amytrophischen Lageralsklerose, die eine nichtspezifische Behandlung bekamen, bestand aus 30 Personen (I9 Männer und II Frauen) im Alter von 24 bis 68 Jahren mit einer Dauer der Erkrankung von 4 Monaten bis zu 5 Jahren. Der Schweregrad der Schädigung des zentralen Nervensystems bei Personen der Kontrollgruppe hat sich wesentlich von dem Schweregrad bei Patienten unterschieden, die mit dem erfindungsgemässen Präparat behandelt wurden. I8 Patienten hatten generalisierte Form, 5 - cervicothoracius, 4- lumbosacralis, 3 - bulbäre Form der amyotrophischen Lateralsklerose.

Die Untersuchungsergebnisse des Einflusses des erfindungsgemässen Präparates auf den Verlauf der amyotrophischen Lateralsklerose sind in der Tabelle 4 angeführt.

Wie aus der Tabelle zu ersehen ist, wurden im Ergebnis der durchgeführten Behandlung mit dem erfindungsgemässen Präparat bei den meisten Patienten - 52 Personen (84%) - keine Anzeichen einer sich verschlimmernden Entwicklung nachgewiesen. Die sich verschlimmernde Entwicklung der amyotrophischen Lateralsklerose wurde lediglich bei IO Personen (I6%) beobachtet. Dabei blieb der Zustand bei 28 Personen (45%) stabil und bei 24 Personen (39%) war die Verbesserung des Zustandes festgestellt.

Positive Veränderungen im neurologischen Status der Patienten mit der amyotrophischen Lateralsklerose zeichneten sich durch das Ansteigen der Kraft in den gelähmten Extremitäten aus, die zur Vergrösserung des Umfanges der Bewegungen, zur Verringerung des Ausprägegrades bulbärer Störungen, darunter Schluckstörungen und Dysarthrie führte. Bei einigen Patienten wurde die Verbesserung der Atmungskapazität der Lunge um IO bis I5% festgestellt. Bei 8 Personen (I2,9%) wurde ein bedeutender Grad der Verbesserung nachgewiesen, der in Erweiterung von Bewegungsfertigkeiten zum Ausdruck kam; infolgedessen wurde es mög-

- I6 -

lich, selbständig sich zu setzen, zu sitzen und sogar sich selbständig innerhalb des Krankenhauses zu bewegen.

Als ein wichtiger Umstand soll hervorgehoben werden, dass auf dem Hintergrund der Einführung des erfindungsgemässen Präparates bei der überwiegenden Mehrheit von Patienten die Verringerung des Asprägegrades depressiver Reaktionen festgestellt wurde.

Positive Änderungen waren vorwiegend bei Patienten mit relativ kürzerer Dauer der Erkrankung (bis zu 2 Jahren) und mit dem Vorherrschen der klinischen Symptome der Schädigung von Pyramidenstrukturen und der cervicothoracius Abteilung des Rückenmarks zu beobachten.

Bei den Patienten der Kontrolgruppe, denen die allgemeingültige komplexe Therapie der amyotrophischen Lateralsklerose verordnet wurde, war in den meisten Fällen, bei 23 Personen (77%) die sich verschmlimmernde Entwicklung des pathologischen Prozesses zu verzeichnen. Lediglich bei 6 Personen (20%) wurde stabiler Zustand beobachtet. Bei einem Patienten (3%) wurde die Verbesserung des neurologischen Symptomzustandes festgestellt, der eine gewisse Vergrösserung des Umfangs der Bewegungen in den Beinen und die Möglichkeit des Gehens infolge der Verringerung des spastischen Tonus in den Beinmuskulatur brachte. In keinem einzigen Fall wurde eine ausgeprägte Verbesserung mit der Vergrösserung der Bewegungsfertigkeiten beobachtet.

Tabelle 4

Einfluss des Präparates und der nichtspezifischen Therapie auf den Verlauf der amyotrophischen Lateralsklerose

| Verfahren der Behandlung | Anzahl der Patienten | Verbesserung | | Fehlen der Dynamik | Sich verschlimmernde Entwicklung |
|---|---|---|---|---|---|
| | | Ausgeprägt | Mässig | | |
| 1 | 2 | 3 | 4 | 5 | 6 |
| Präparat + Nichtspezifische Therapie | 62 (I00%) | 8 (I3%) | I6 (26%) | 28 (45%) | I0 (I6%) |
| Nichtspezifische Therapie | 30 (I00%) | – | I (3%) | 6 (20%) | 23 (77%) |

- I7 -

Hierdurch zeigte die vergleichende Analyse des Zustandes der beiden Gruppen von Patienten (Behandlung mit dem erfindungsgemässen Präparat in Verbindung mit dem allgemeingültigen Komplex von Arzneimitteln und die Behandlung nur mit diesem Komplex von Mitteln), dass unter den Patienten, die das beanspruchte Präparat einnahmen, die sich verschlimmernde Entwicklung wesentlich seltener im Vergleich zur Kontrollgruppe (um das 4,8fache) festgestellt wurde. Bei den Personen, denen das erfindungsgemässe Präparat eingeführt wurde, entwickelte sich der stabile Zustand oder die Verbesserung des Zustandes in 84% der Fälle und bei den Personen der Kontrollgruppe in 23% der Fälle. Während bei I3% der Patienten unter der Einwirkung des erfindungsgemässen Präparates eine ausgeprägte Verbesserung des Zustandes unter Zunahme der Bewegungsfertigkeiten zu verzeichnen war, wurde in der Kontrollgruppe solche Verbesserung in keinem einzigen Fall beobachtet.

Die erzielten Angaben zeugen davon, dass das erfindungsgemässe Präparat bei der Therapie der amyotrophischen Lateralsklerose zu einer Verlangsamung der Verschlimmerung des pathologischen Prozesses und zur Verbesserung des Zustandes der Patienten beiträgt.

Somit zeigt das erfindungsgemässe Präparat eine hohe Effektivität bei der Behandlung solcher schwerer neurologischer Erkrankungen wie die multiple Sklerose und die myotrophische Lateralsklerose. Bei der Behandlung der multiplen Sklerose mit dem erfindungsgemässen Präparat wurde klinische Verbesserung bei 89% der Patienten, und bei Personen, die mit T-Aktivin behandelt wurden, lediglich bei 56% der Fälle festgestellt. Dabei war die Effektivität des erfindungsgemässen Präparates von dem Schweregrad und der Dauer der Erkrankung nicht abhängig. Bei der Behandlung der multiplen Sklerose wurde bei dem beanspruchten Präparat eine ganz neue Eigenschaft nachgewiesen, die Fähigkeit, den funktionellen Zustand der Leitbahnen des Hirnstammes zu verbessern.

Eine hohe Effektivität zeigt das beanspruchte Prä-

parat bei Therapie der Erkrankung der amyotrophischen Lateralsklerose, die keine pathogenetische und spezifische Therapie hat und die sich durch eine unentwegte Verschlimmerung mit einem schnellen tödlichen Ausgang auszeichnet. Wenn bei der Anwendung der allgemeingültigen nichtspezifischen Therapie die Stabilisierung des Prozesses beziehungsweise eine klinische Verbesserung bei 23% der Patienten erreicht wurde, so traten die Stabilisierung beziehungsweise klinische Verbesserung bei der Einbeziehung des angemeldeten Präparates in diesen Komplex bei 84% der Patienten ein, das heisst bei der absoluten Mehrheit, bei einigen Patienten wurde ein wesentlicher Grad der Verbesserung erreicht, der in der Erweiterung von Bewegungsfertigkeiten zum Ausdruck kam.

Bei der Behandlung mit dem Präparat wird keine Entwicklung von unerwünschten Nebenreaktionen nachgewiesen. Die Arzneiformen des beanspruchten Präparats werden in an sich bekannter Weise hergestellt.

Der Wirkstoff des erfindungsgemässen Präparates kann in Standard-Verfahren der Peptidchemie synthetisiert werden, insbesondere im Verfahren eines stufenweisen Wachsens einer Peptidkette, beginnend mit C-endigem freiem Arginen, mit Hilfe aktivierten Estern der geschützten Aminosäuren durch folgende Zwischenverbindungen: Karbobenzoxy-leuzyl-arginen; Karbobenzoxy-phenylalanyl-leuzyl-arginen; Karbobenzoxy-glyzyl-phenyl-alanyl-leuzyl-arginen; Karbobenzoxy-D-alanyl-glyzyl-phenyl-alanyl-leuzyl-arginen; Karbobenzoxy-O-benzyl-tyrosyl-D-alanyl-glyzyl-phenylalanyl-leuzyl-arginin.

Zur besseren Erläuterung der vorliegenden Erfindung werden nachstehende konkrete Beispiele der klinischen Erprobung des erfindungsgemässen Präparates, die die Effektivität seiner Anwendung veranschaulichen, angeführt.

Beispiel I

Patientin K., 46 Jahre alt, mit einer Diagnose: multiple Sklerose, zerebrospinale Form, progredienter Verlauf. Die Dauer der Erkrankung 27 Jahre. Der Verlauf der Erkrankung mit Verschlimmerungen und Remissionen, unentwegt sich verschlimmernde Entwicklung der Erkrankung, Invalidin der I. Gruppe.

Neurologischer Status bei der Einlieferung: Senkung des Sehevermögens, Glättung der nasolabialen Falte von rechts, Nystagmus, Schwierigkeiten beim Schlucken der Nahrung, untere spastische Paraparese mit Einschränkung der Bewegung der oberen Sprunggelenke und in Kniegelenken, beiderseitiger Klonus der Füsse, pathologische Pyramidenanzeichen, kann nicht gehen, Muskelkraft der Hände ist bis zu 3 bis 4 Punkten gesunken, beiderseitige Symptome von Jacobson und Rossolimo, ausgeprägte Intention bei der Ausführung von Koordinationsproben, Rumpfataxie, Störungen der tiefen Empfindlichkeit an den Füssen. Die Gesamtpunktenzahl des neurologischen Defizits beträgt I8 und nach der Skale der sich verschlimmernden Entwicklung - 6. Bei der Untersuchung der kurzlatenten erzeugten Hörpotentiale sind Anzeichen der Schädigung des Hirnstammes vorhanden, Elektroenzephalogramm ist von disynchronem Typ.

Bei der Einlieferung ins Krankenhaus wurden der Patientin Vitamin $B_I$, Glutaminsäure, Midokalm, kurzfristige Einnahmen von Pyrazetam und Essentiale verordnet. Dann wurde die Patientin innerhalb von 5 Tagen mit dem angemeldeten Präparat behandelt, das man in einer einmaligen Dosis von I mg zweimal pro Tag einführte.

Nach der Kur mit dem angemeldeten Präparat stieg die Kraft in den Beinen, es verringerte sich der Ausprägegrad der Kleinhirnschädigungen und der Symptome der Schädigung des Hirnstammes. Die Gesamtpunktenzahl des neurologischen Defizits betrug IO, nach der Skale der sich verschlimmernden Entwicklung - 4. Die Patientin begann mit Unterstützung zu gehen, zu verzeichnen war eine positive Dynamik seitens des Elektroenzephalogramms und der kurzlatenten erzeugten Hörpotentiale.

Beispiel 2

Patient K, im Alter von 20 Jahren, mit einer Diagnose: multiple Sklerose, zerebrospinale Form, remittierender Verlauf, im Stadium der Remission. Die Dauer der Erkrankung betrug 6 Jahre, Beginn der rechtsseitigen Hemi-

parästhesie. Später der wellenartige Verlauf der Erkrankung unter Anschluss von Kleinhirnsymptomen, des unteren spastischen Paraparese, Hirnstammsymptomen, Senkung des Sehvermögens, Schädigungen der Funktionen der Beckenorgane. Invalide der III. Gruppe.

Neurologischer Status bei der Einlieferung: Senkung des Sehevermögens am rechten Auge, Diplopie beim Blicken nach rechts, Nystagmus, untere spastische Paraparese mit Verringerung der Muskelkraft bis zu 3 Punkten, beiderseitige Verstärkung der Sehnenreflexe mit pathologischen Pyramidenzeichen, Rumpfataxie, Intention bei der Ausführung von Koordinationsproben, bewegt sich nur mit fremder Hilfe, Störung der Funktion der Beckenorgane, Störung der Vibrationsempfindlichkeit in rechten Extremitäten. Die Gesamtpunktenzahl des neurologischen Defizits beträgt I6 Punkte, nach der Skale der sich verschlimmernden Entwicklung - 5. Bei der Untersuchung der kurzlatenten erzeugten Hörpotentiale sind Anzeichen der Schädigung des Hirnstammes vorhanden. An einem Elektroenzephalogramm sind wesentliche diffuse Änderungen zu sehen.

Bei der Einlieferung ins Krankenhaus wurden dem Patienten Vitamine $B_I$ und $B_6$, Glutaminsäure, innerhalb eines Monates Zerebrolysin verabreicht, durchgeführt wurden kurze Kuren mit Midokalm und Essentiale. Dann wurde der Patient innerhalb von 5 Tagen mit dem erfindungsgemässen Präparat behandelt, das man intramuskulär in einer einmaligen Dosis von 2 mg zweimal am Tage einführte.

Nach der Kur mit dem erfindungsgemässen Präparat waren die Kleinhirnschädigungen vollständig und teilweise die Bewegungsstörungen verschwunden, steht sicher und selbständig, keine Sensorstörungen, wesentliche Verringerung der Diplopie. Am Elektroenzephalogramm und bei kurzlatenten erzeugten Hörpotentialen eine positive Dynamik. Die Gesamtpunktenzahl des neurologischen Defizits betrug 8 Punkte, nach der Skale der sich verschlimmernden Entwicklung - 3 Punkte.

Beispiel 3

- 2I -

Patient G., im Alter von 50 Jahren, mit der Diagnose: amyotrophische Lateralsklerose. Zunächst entstanden Sprachstörungen, nach einigen Monaten kamen noch Schluckstörungen dazu. Der Zustand verschlimmerte sich weiterhin. Nach Ablauf eines Jahres entwickelte sich die Schwäche in Beinen und im linken Arm sowie Atmungsbeschwerden.

Bei der Einlieferung im neurologischen Status: Anarthrie, Zunge ist in der Mundhöhle unbeweglich, Dysphonie, selbständig kann nicht schlucken, der weiche Gaumen ist unbeweglich, Schwäche der Gesichtsmuskulatur. Tetraparese (am meisten in Händen) mit Einschränkungen der Bewegung in Schultergelenken (bis 4 Punkten). Verringerung der Kraft in Beinen (2 Punkte), mit Schwierigkeit bewegt sich im Krankenzimmer. Generalisierte Hypertrophie der Muskel und Faszikulation in denselben. Sehnenreflexe sind hoch, beiderseitige pathologische Zeichen. Empfindlichkeit und Funktionen der Beckenorgane sind nicht gestört. Atmung ist oberflächlich, beschleunigt, Hustenkrampf herabgesetzt. Lebenskapazität der Lungen betrut I,32 1 (30%, bezogen auf die Norm).

Im Krankenhaus wurde der Patient mit Vitaminen der B-Gruppe, Finlepsin, Desoxyribonuklease und Midokalm behandelt. Unter Anwendung dieser Behandlung führte man dem Patienten innerhalb von 2 Wochen intramuskulär zweimal am Tage je I mg des erfindungsgemässen Präparates, das in 0,9%iger wässeriger Lösung des Natriumchlorids verdünnt ist, ein. Nach einer IO-tägigen Unterbrechung wurde die Behandlung mit dem erfindungsgemässen Präparat wiederholt.

Infolge der durchgeführten Behandlung wurde bei dem Patienten die Kraft in den Händen grösser (die Parese verringerte sich bis auf I Punkt) und in den Füssen (Parese O Punkte), er konnte ausserhalb des Krankenhauses sich bewegen und Treppen steigen. Es entstand ausserdem die Möglichkeit, geringe selbständige Schluckbewegungen auszuführen.

Beispiel 4

Patient E., im Alter von 4I Jahren, mit einer Diag-

- 22 -

nose: amyotrophische Lateralsklerose. Ursprünglich trat Heiserkeit der Stimme und in 2 bis 3 Monaten Schwäche im linken Fuss auf. Später entwickelte sich die Schwäche in Armen mit Schmerzen im rechten Arm, Atrophie der Muskel der Schultergegend, der Hände, verbreitete Muskelzuckungen. Diese Erscheinungen entwickelten sich weiter, im letzten Halbjahr kamen die Schwäche in den Beinen und erschwerte Atmung dazu.

Im neurologischen Status bei der Einlieferung: horizontaler Nystagmus, Dysarthrie, klanglose Stimme, kann ohne Unterbrechung nur während 5 bis 7 Minuten sprechen. Beim Essen verschluckt sich. Parese des weichen Gaumens. Schwäche der Gesichtsmuskulatur, Tetraparese, besonders ausgeprägt in distalen Abschnitten der Beine (bis zu 4 Punkten). Zu verzeichnen ist die Fibrillation der Zungenmuskel. Das Gehen ist erschwert, die Treppen kann nicht steigen. Sehnenreflexe sind herabgesetzt. Empfindlichkeit und Funktionen der Beckenorgane sind nicht gestört.

Im Krankenhaus wurde der Patient mit Vitaminen $B_6$ und $B_{12}$, mit Vitamin E, Finlepsin, Baklofen und Retabolyl behandelt. Unter Anwendung dieser Behandlung wurde dem Patienten innerhalb von 2 Wochen, intramuskulär, zweimal am Tage je 2 mg des erfindungsgemässen Präparates, das in 0,9%iger wässeriger Lösung des Natriumchlorids verdünnt war, eingeführt. Nach einer 10-tägigen Unterbrechung wurde die Kur mit dem erfindungsgemässen Präparat wiederholt.

Infolge der durchgeführten Behandlung verbesserte sich der Zustand des Patienten: grösser wurde die Kraft in paroximalen Abschnitten von Beinen und Armen (Paresegrad 0 Punkte), er konnte schon Treppen steigen. Die Stimme wurde klangvoller, es verbesserte sich etwas die Beweglichkeit des weichen Gaumens, die Dysarthrie verringerte sich, hierdurch kann der Patient ohne Erholung bedeutend länger sprechen und sich praktisch bei einem gewöhnlichen Gespräch nicht einschränken.

Beispiel 5.

Patient M., im Alter von 33 Jahren, mit einer Diag-

nose: amyotrophische Lateralsklerose. Ursprünglich trat die Schwäche in Beinen beim Gehen auf. In 5 Monaten trat die Schwäche in Armen auf, dann entstanden Schwierigkeiten beim Sprechen. Der Zustand verschlechterte sich fortschreitend. Im neurologischen Status bei der ersten Einlieferung: Dysarthrie, Parese des weichen Gaumens, Tetraparese, besonders ausgeprägt in Armen (4 Punkte), Hypotrophie der Muskel der Arme, der Schulter, hohe Sehnenreflexe, beiderseitige pathologische Zeichen, verbreitete Faszikulationen, Symptome des oralen Automatismus. Empfindlichkeit und Funktionen der Beckenorgane sind nicht gestört.

Im Krankenhaus wurde der Patient mit Vitaminen $B_I$ und $B_{I2}$, Pyrazetam, Retabolyl, Finlepsin und Ribonukleinsäure behandelt. Unter Anwendung dieser Behandlung wurden dem Patienten innerhalb von 2 Wochen, intramuskulär, zweimal am Tage je I mg des erfindungsgemässen Präparates, das in 0,9%iger wässeriger Lösung des Natriumchlorids verdünnt war, eingeführt.

Infolge der durchgeführten Behandlung verbesserte sich der Zustand des Patienten, was in der Vergrösserung der Kraft in Beinen (Parese verringerte sich von 3 Punkten bis I Punkt) zum Ausdruck kam.

Nach Entlassung hat der Patient keine Verschlechterung des Zustandes innerhalb von 2 bis 3 Monaten empfunden, dann trat das Ansteigen der Sprechstörungen und der Schwäche in den Armen auf (die Verschlechterung war mit einer kraniozerebralen Verletzung verbunden). Bei der wiederholten Einlieferung ins Krankenhaus wurde festgestellt: Dysarthrie, Dysphagie, Fibrillation der Zungenmuskel. Der Patient konnte lediglich 2 bis 3 Minuten ohne Erholung sprechen. Tetraparese am meisten in den Armen und vorwiegend in den distalen Abschnitten (bis 4 Punkten), mit beschränkter Möglichkeit des Zusammenballens der Finger und ihrer Spreizung, sowie gelang es ihm nicht, den ersten Finger dem fünften Finger rechts entgegenzustellen. An Händen war Parese bis zu 2 Punkten, das Gehen ohne Erholung war nur für eine Entfernung

- 24 -

bis 300 m möglich. Hypotrophie der Muskel der Schultergegend und der Arme, ausgebreitete Faszikulationen. Zu verzeichnen sind hohe Sehnenreflexe. Empfindlichkeit ist nicht gestört. Funktionen der Beckenorgane sind nicht verändert.

Unter Anwendung der Therapie mit Vitaminen der B-Gruppe, mit Vitamin E, Pyrazetam, Finlepsin, Baklofen führte man dem Patienten innerhalb von 2 Wochen, intramuskulär, zweimal am Tage je I mg des erfindungsgemässen Präparates, das in einer 0,9%igen wässerigen Lösung des Natriumchlorids verdünnt ist, ein. Nach einer IO-tägigen Unterbrechung wurde die Kur mit dem erfindungsgemässen Präparat wiederholt.

Infolge der durchgeführten Behandlung wurde das Ansteigen der Kraft in den Extremitäten beobachtet. Parese sank bis 0 Punkte, möglich wurde das Gehen ohne Erholung auf eine Entfernung bis I,5 km. Die Kraft in den Händen wurde grösser, möglich wurde das Zusammenballen der Finger und ihre Spreizung. Dysarthrie verringerte sich, der Patient konnte ohne Erholung bedeutend länger sprechen, ohne sich praktisch bei einem gewöhnlichen Gespräch einzuschränken.

Gewerbliche Anwendbarkeit

Das erfindungsgemässe Präparat findet in der Neurologie als ein Arzneimittel für die Behandlung der multiplen Sklerose und der myotrophischen Lateralsklerose seine Anwendung.

- 25 -

PATENTANSPRÜCHE

I. Arzneimittel für Behandlung der multiplen Sklerose und der amyotrophischen Lateralsklerose, das einen Wirkstoff und ein pharmazeutisches Verdünnungsmittel enthält, dadurch g e k e n n z e i c h n e t, dass es als Wirkstoff ein Peptid folgender Struktur aufweist:

Tyr-D-Ala-Gly-Phe-Leu-Arg.

2. Arzneimittel nach Anspruch I in Form einer Injektionslösung, dadurch g e k e n n z e i c h n e t, dass es den Wirkstoff in einer Menge von I bis 5 mg/ml enthält.

3. Arzneimittel nach Anspruch I-2, dadurch g e - k e n n z e i c h n e t, dass es als pharmazeutisches Verdünnungsmittel ein Lösungsmittel, 0,9%ige wässerige Natriumchloridlösung enthält.

4. Arzneimittel nach Anspruch I in Form von Tabletten, dadurch g e k e n n z e i c h n e t, dass es den Wirkstoff in einer Menge von IO bis I5 mg je eine Tablette enthält.

5. Arzneimittel nach Anspruch I und 4, dadurch g e k e n n z e i c h n e t, dass es als pharmazeutisches Verdünnungsmittel eine Trägersubstanz für Tabletten, Stärke, Glukose beziehungsweise Laktose, enthält.

6. Arzneimittel nach Anspruch I in Form eines intranasalen Aerosols, dadurch g e k e n n z e i c h n e t, dass es den Wirkstoff in einer einmaligen Dosis von I bis 5 mg enthält.

# INTERNATIONAL SEARCH REPORT

0336976

International Application No PCT/SU 88/00162

## I. CLASSIFICATION OF SUBJECT MATTER (if several classification symbols apply, indicate all) [6]

According to International Patent Classification (IPC) or to both National Classification and IPC

IPC$^4$ — A 61 K 37/02

## II. FIELDS SEARCHED

### Minimum Documentation Searched [7]

| Classification System | Classification Symbols |
|---|---|
| IPC$^4$ | A 61 K 37/02 |

### Documentation Searched other than Minimum Documentation to the Extent that such Documents are Included in the Fields Searched [8]

## III. DOCUMENTS CONSIDERED TO BE RELEVANT [9]

| Category [*] | Citation of Document, [11] with indication, where appropriate, of the relevant passages [12] | Relevant to Claim No. [13] |
|---|---|---|
| A | WO,A1,86/00622,(VSESOJUZNY KARDIOLOGICHESKY TSENTR AKADEMII MEDITSINSKIKH NAUK SSSR)30 January 1986(30.01.86),see the abstract | 1-6 |
| A | WO,A1,84/02470,(VSESOJUZNY KARDIOLOGICHESKY TSENTR AKADEMII MEDITSINSKIKH NAUK SSSR) 05 July 1984 (05.07.84),see the claims cited in the description &, GB,A, 2141627, (03.01.85) | 1-6 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 20 December 1988(20.12.88) | 28 December 1988(28.12.88) |

| International Searching Authority | Signature of Authorized Officer |
|---|---|
| ISA/SU | |

Form PCT/ISA/210 (second sheet) (January 1985)